# EUROPÄISCHE PATENTSCHRIFT

(11) **EP 2 651 377 B1**
(45) Veröffentlichungstag und Bekanntmachung des Hinweises auf die Patenterteilung: **12.04.2017**
(21) Anmeldenummer: 11788138.3
(22) Anmeldetag: 24.11.2011
(51) Int. Cl.: A61K 8/36, A61K 8/37, A61K 8/49, A61K 8/60, A61Q 5/08, A61Q 5/10

(54) **AUFHELLMITTEL MIT ACYLPYRIDINIUMVERBINDUNGEN UND BESTIMMTER TENSIDKOMBINATION**
BRIGHTENING AGENT COMPRISING ACYLPYRIDINIUM COMPOUNDS AND DEFINED SURFACTANT COMBINATION
AGENT ÉCLAIRCISSANT CONTENANT DES COMPOSÉS D'ACYLPYRIDINIUM ET UNE COMBINAISON DÉTERMINÉE DE TENSIOACTIFS

(30) Priorität: 17.12.2010 DE 102010063373
(43) Veröffentlichungstag der Anmeldung: 23.10.2013
(73) Patentinhaber: Henkel AG & Co. KGaA, 40589 Düsseldorf (DE)
(72) Erfinder: GROSS, Wibke, 41836 Hückelhoven (DE); NEMITZ, Ralph, 41363 Jüchen (DE); KROOS, Astrid, 40789 Monheim (DE); JANSSEN, Frank, 41470 Neuss (DE); GÜNTHER, Katja, 40721 Hilden (DE); KLEEN, Astrid, 20457 Hamburg (DE); MANNECK, Hartmut, 23860 Klein Wesenberg (DE)
(86) Internationale Anmeldenummer: PCT/EP2011/070884
(87) Internationale Veröffentlichungsnummer: WO 2012/079953

(56) Entgegenhaltungen:
- EP-A2- 2 295 027
- WO-A1-2009/135700
- WO-A2-2010/029006
- WO-A2-2010/054981
- DE-A1-102009 003 155

## Beschreibung

Die vorliegende Erfindung betrifft Mittel zum Aufhellen keratinischer Fasern, insbesondere menschlicher Haare, enthaltend einen speziellen Bleichaktivator und spezielle oberflächenaktive Verbindungen.

Die Veränderung von Haarfarbe stellt einen wichtigen Bereich der modernen Kosmetik dar. Für den Zweck der Aufhellung der eigenen Haarfarbe bzw. für das Blondieren sind im Markt verschiedene Blondiermittel mit unterschiedlicher Blondierleistung erhältlich. Die Grundlagen der Blondierverfahren sind dem Fachmann bekannt und können in einschlägigen Monographien, z. B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Die in Blondiermitteln enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen eingesetzt. Mit der Aufhellung geht jedoch auch eine Schädigung des Haares einher, da nicht nur die natürlichen farbgebenden Komponenten des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Mit größeren Mengen an Wasserstoffperoxid und gegebenenfalls an Peroxodisulfaten gehen in der Regel stärkere Schädigungen der Keratinfaser einher. Auch wenn die bislang auf dem Markt befindlichen Blondiermittel in der Regel gute Aufhellleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden. Es besteht daher weiterhin Bedarf nach Aufhellmitteln, welche eine gute Aufhellleistung zeigen, ohne gleichzeitig die Haarfaser zu schädigen.

Speziell bei der mehrmaligen Durchführung von Blondierungen können unterschiedliche Haarpartien unterschiedlich stark geschädigt werden. Unterschiedlich starke Vorschädigungen in verschiedenen Bereichen des Haares sind oftmals die Ursache für eine verschieden starke Penetrationsfähigkeit der Aktivsubstanzen eines Blondiermittels in die Haarfaser hinein. Somit können die in Blondiermitteln enthaltenen Alkalisierungsmittel und Oxidationsmittel leichter in den stärker geschädigten Spitzenbereich hinein diffundieren, wodurch in diesem Bereich im Vergleich zum weniger geschädigten Ansatz eine stärkere Blondierwirkung auftritt. Dies führt zu einer unvorteilhaften Ungleichmäßigkeit des Aufhellergebnisses und ist vom Verbraucher nicht erwünscht.
Aus diesem Grund besteht ein aktueller Bedarf an optimierten Mitteln zum Blondieren von Haaren, welche eine über den bislang bekannten Stand der Technik hinausragende Aufhellleistung zeigen, die Haare hierbei nach Möglichkeit nicht schädigen und gleichzeitig ein gleichmäßiges, attraktives Aufhellergebnis liefern.
Eine vom Verbraucher stark negativ wahrgenommene Auswirkung der im Zuge eines Blondiervorgangs auftretenden Haarschädigung ist der Griff des Haares, welcher sich von einem ursprünglich glatten Griffgefühl in Richtung einer mehr strohigen taktilen Wahrnehmung verändert.

Die Blondierung von Haaren unter Zuhilfenahme einer Kombination von kationischen Acylpyridiniumderivaten und Wasserstoffperoxid in Kombination mit bestimmten Coaktivatoren ist aus der WO2009/135700 A1 bekannt. Damit soll eine Verstärkung des Aufhell-Effektes erzielt werden.
In WO 2010/054981 A2 wird der Einsatz von kationischen Pyridiniumderivaten in Kombination mit Farbstoffen beschrieben. WO 2010/029006 A2 beschreibt Aufhellmittel mit 2-Acylpyridiniumderivaten. DE 10 2009 003 155 A1 adressiert die Haarpflege, die bei Einsatz von Acetylpyridiniumsalzen erzielt werden kann.
Dem Stand der Technik war bisher nicht zu entnehmen, dass eine Kombination von bestimmten Acylpyridiniumderivaten und Wasserstoffperoxid nicht nur, wie bereits bekannt, die Aufhellleistung im Vergleich zu einer normalen Blondierung, enthaltend Wasserstoffperoxid verbessert, sondern auch zu einem angenehmeren Griff der behandelten Haare führt.
Es ist daher die Aufgabe der vorliegenden Erfindung, diese negative Wahrnehmung durch Optimierung des Blondiermittels so weit wie möglich zu reduzieren.

Im Zuge der zu dieser Erfindung führenden Arbeiten hat sich nun überraschenderweise herausgestellt, dass die Pflegewirkung des erfindungsgemäßen Blondiermittels, insbesondere das Griffgefühl der Haare, durch Einsatz einer speziellen Kombination aus Tensiden weiter verbessert werden kann.

Ein erster Gegenstand der Erfindung ist daher ein Mittel zum Aufhellen von keratinischen Fasern, welches in einem kosmetischen Träger mindestens ein anionisches, Carbonsäure-haltiges Tensid, mindestens ein nichtionisches Tensid, und mindestens Wasserstoffperoxid, dadurch gekennzeichnet, dass das Mittel zusätzlich mindestens ein Acylpyridiniumderivat der Formel (I), 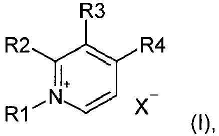 worin
- R1: für eine C₁-C₆-Alkylgruppe, eine C₂-C₆-Alkenylgruppe, eine C₂-C₆-Hydroxyalkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
- R2, R3 und R4: jeweils unabhängig voneinander für Wasserstoff, eine C₁-C₆-Alkylgruppe, ein Halogenatom oder eine C₁-C₆-Acylgruppe steht, mit der Massgabe, dass zumindest einer der Reste R2, R3 und R4 für eine C₁-C₆-Acylgruppe steht,
- X⁻: für ein physiologisch verträgliches Anion steht, enthält,
dadurch gekennzeichnet, dass
das Mittel mindestens ein anionisches, Carbonsäure-haltiges Tensid enthält, welches ausgewählt ist aus den Alkylglucosidcarbonsäuren.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.
Die erfindungsgemäßen Mittel enthaltend die Wirkstoffe in einem kosmetischen Träger. Der kosmetische Träger ist bevorzugt wässrig, alkoholisch oder wässrig-alkoholisch. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Ein wässriger Träger enthält im Sinne der Erfindung mindestens 40 Gew.-%, insbesondere mindestens 50 Gew.-% Wasser. Unter wässrigalkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.
Als ersten wesentlichen Inhaltstoff enthalten die erfindungsgemäßen Mittel mindestens ein Acylpyridiniumderivat gemäß Formel (I). Beispiele für die als Substituenten der Verbindungen der Formel (I) genannten werden nachfolgend, aber nicht beschränkend genannt: Beispiele für C₁-C₆-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, - CH(CH₃)CH₂CH₃, -C(CH₃)₃. Beispiele für eine C₂-C₆-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enylgruppe bevorzugt ist. Beispiele für eine C₂-C₆-Hydroxyalkylgruppe sind -CH₂CH₂OH, -CH₂CH₂CH₂OH, -CH₂CH(OH)CH₃ und-CH₂CH₂CH₂CH₂OH, wobei die Gruppe -CH₂CH₂OH bevorzugt ist. Beispiele für C₁-C₆-Alkoxy-C₂-C₆-alkylgruppen sind die Gruppen -CH₂CH₂OCH₃, -CH₂CH₂CH₂OCH₃, -CH₂CH₂OCH₂CH₃, - CH₂CH₂CH₂OCH₂CH₃, -CH₂CH₂OCH(CH₃)₂, -CH₂CH₂CH₂OCH(CH₃)₂. Beispiele für eine Carboxy-C₁-C₆-alkylgruppe sind die Carboxymethylgruppe, die 2-Carboxyethylgruppe oder die 3-Carboxypropylgruppe. Beispiele für Aryl-C₁-C₆-alkylgruppen sind die Benzylgruppe und die 2-Phenylethylgruppe. Beispiele für eine Heteroaryl-C₁-C₆-alkylgruppe sind die Pyridin-2-ylmethylgruppe, die Pyridin-3-ylmethylgruppe, die Pyridin-4-ylmethylgruppe, die Pyrimidin-2-ylmethylgruppe, die Pyrrol-1-ylmethylgruppe, die Pyrrol-1-ylethylgruppe, die Pyrazol-1-ylmethylgruppe oder die Pyrazol-1-yl-ethylgruppe. Beispiele für eine Arylgruppe sind die Phenylgruppe, die 1-Naphthylgruppe oder die 2-Naphthylgruppe. Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-4-ylgruppe. Beispiele einer C₁-C₆-Acylgruppe sind Acetyl (1-Oxo-ethyl), 1-Oxo-propyl, 1-Oxo-butyl, 1-Oxo-Pentyl, 1-Oxo-2,2-dimethylpropyl und 1-Oxo-hexyl.

In einer Ausführungsform der vorliegenden Erfindung sind solche Verbindungen gemäß Formel (I) bevorzugt, bei welchen der Rest R1 der allgemeinen Struktur (I) für eine C₁-C₆-Alkylgruppe, für eine C₂-C₆-Alkenylgruppe oder für eine C₂-C₆-Hydroxyalkylgruppe steht. Es ist erfindungsgemäß bevorzugt, wenn der Rest R1 für eine C₁-C₆-Alkylgruppe, bevorzugt für Methyl, Ethyl, n-Propyl oder Isopropyl und insbesondere bevorzugt für Methyl, steht.

Es hat sich herausgestellt, dass die Acylpyridiniumderivate gemäß Formel (I) erfindungsgemäß besonders vorteilhafte Eigenschaften besitzen, wenn sie die Acyl-Gruppe entweder in 2- oder 4-Position am Pyridin-Ring tragen. Bevorzugte Verbindungen der Formel (I) sind weiterhin solche Verbindungen, bei denen entweder der Rest R2 oder der Rest R4 für eine C₁-C₆-Acylgruppe, bevorzugt für eine Acetylgruppe, steht. Es ist weiterhin bevorzugt, wenn einer der Reste R2 oder R4 für eine Acetylgruppe steht, während der andere dieser Reste sowie der Rest R3 jeweils für Wasserstoff stehen. Eine weitere Ausführungsform der vorliegenden Erfindung ist daher dadurch gekennzeichnet, dass das Mittel als Acylpyridiniumderivat gemäß Formel (I) mindestens ein 2-Acetylpyridiniumderivat und/oder 4-Acetylpyridiniumderivat enthält. Geeignete Acetylpyridiniumderivate sind dabei insbesondere die physiologisch verträglichen Salze, die als Kation ein Acetylpyridiniumderivat, ausgewählt aus 4-Acetyl-1-methylpyridinium, 4-Acetyl-1-allylpyridinium, 4-Acetyl-1-(2-hydroxyethyl)pyridinium, 2-Acetyl-1-methylpyridinium, 2-Acetyl-1-allylpyridinium und 2-Acetyl-1-(2-hydroxyethyl)pyridinium, enthalten.

Es ist bevorzugt, wenn das Anion X⁻ gemäß Formel (I) ausgewählt wird aus Halogenid, insbesondere Chlorid, Bromid und lodid, Benzolsulfonat, p-Toluolsulfonat, C₁-C₄-Alkylsulfonat, Trifluormethansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß vorteilhaft ist es, wenn das physiologisch verträgliche Anion X⁻ für ein Halogenidion (insbesondere Chlorid oder Bromid), Hydrogensulfat, p-Toluolsulfonat, Benzolsulfonat oder Acetat steht.

Insbesondere sind solche Mittel erfindungsgemäß bevorzugt, die dadurch gekennzeichnet sind, dass das Acylpyridiniumderivat gemäß Formel (I) ausgewählt ist aus mindestens einer Verbindung der Gruppe, die gebildet wird aus 4-Acetyl-1-methylpyridinium-p-toluolsulfonat, 4-Acetyl-1-methyl-pyridinium-benzolsulfonat, 4-Acetyl-1-methylpyridiniumhydrogensulfat, 4-Acetyl-1-methylpyridinium-acetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridinium-acetat. Erfindungsgemäß besonders bevorzugte Mittel sind dadurch gekennzeichnet, dass sie als das Acylpyridiniumderivat gemäß Formel (I) eine Verbindung, ausgewählt aus 4-Acetyl-1-methylpyridinium-p-toluolsulfonat und/oder 2-Acetyl-1-methylpyridinium-p-toluolsulfonat, insbesondere 4-Acetyl-1-methylpyridinium-p-toluolsulfonat, enthalten.

Eine Ausführungsform der vorliegenden Erfindung ist dabei dadurch gekennzeichnet, dass im erfindungsgemäßen Mittel die Acylpyridiniumderivate der Formel (I) in einem Gewichtsanteil von 0,1 bis 10 Gew.-%, insbesondere von 0,2 bis 4 Gew.-%, und insbesondere 0,5 bis 2 Gew.-%, jeweils bezogen das Gesamtgewicht des Mittels, enthalten sind.

Als weitere wesentliche Inhaltsstoffe enthalten die erfindungsgemäßen Mittel weiterhin mindestens ein anionisches, Carbonsäure-haltiges Tensid und mindestens ein nichtionisches Tensid.

Als Tenside werden erfindungsgemäß grenzflächenaktive Verbindungen oder Emulgatoren bezeichnet, die über wenigstens ein hydrophiles und ein hydrophobes Strukturelement verfügen.

Anionische, Carbonsäure-haltige Tenside sind gekennzeichnet durch eine wasserlöslich machende, anionische Carboxylat-Gruppe und eine lipophile Alkylgruppe mit etwa 8 bis 30 C-Atomen. Zusätzlich können in der Molekülstruktur Glycol- und Polyglycolether-Gruppen, Ester-, Ether- und Amidgruppen sowie Hydroxylgruppen enthalten sein.

In den erfindungsgemäßen Mitteln werden die anionischen, Carbonsäure-haltigen Tenside in der Regel in Form ihrer Natrium-, Kalium- und Ammonium- sowie der Mono, Di- und Trialkanolammoniumsalze mit 2 bis 4 C-Atomen in der Alkanolgruppe eingesetzt oder durch ein vorhandenes Alkalisierungsmittel durch Deprotonierung in ihre Salzform überführt.

Beispiele für solche anionischen Tenside sind, jeweils in Form der Natrium-, Kalium- und Ammoniumsowie der Mono, Di- und Trialkanolammoniumsalze mit 2 bis 4 C-Atomen in der Alkanolgruppe,Alkylglucosidcarbonsäuren.

Geeignete Alkylglucosidcarbonsäuren sind, insbesondere in Form ihrer Natrium- und AmmoniumSalze, Alkylether von Zuckerbausteinen, bei denen wenigstens eine Hydroxyfunktion mit einer OCH₂CO₂H-Einheit ausgetauscht wurde. Ein Beispiel für eine Alkylglucosidcarbonsäure ist Lauryl Glucose Carboxylate (Handelsprodukt: Plantapon LGC Sorb, Cognis; Kombination aus Lauryl Glucose Carboxylate und Lauryl Glucoside).

Eine Ausführungsform des ersten Erfindungsgegenstands ist ein Mittel, welches dadurch gekennzeichnet ist, dass das Mittel mindestens ein anionisches, Carbonsäure-haltiges Tensid enthält, welches ausgewählt ist aus Alkylglucosidcarbonsäuren.

Eine weitere Ausführungsform des ersten Erfindungsgegenstands ist ein Mittel, welches dadurch gekennzeichnet ist, dass das Mittel das und/oder die anionischen, Carbonsäure-haltigen Tenside in einem Gewichtsanteil von 0,5 bis 25 Gew.-%, insbesondere von 1,0 bis 15 Gew.-%, und insbesondere 1,5 bis 10 Gew.-%, bezogen das Gesamtgewicht des anwendungsbereiten Mittels, enthält. Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe.

Solche Verbindungen sind beispielsweise Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; Polyolfettsäureester, insbesondere (Poly)glycerinfettsäureester und alkoxylierte Polyglycerinfettsäureester, wie höher alkoxylierte, propoxylierte und insbesondere ethoxylierte, Mono-, Di-und Triglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylpolyglucoside entsprechend der allgemeinen Formel RO-(Z)ₓ, wobei R für Alkyl, Z für Zucker sowie x für die Anzahl der Zuckereinheiten steht.

Anlagerungsprodukte von 1 bis 50 Mol Ethylenoxid und/oder 0 bis 5 Mol Propylenoxid an lineare und verzweigte Fettalkohole mit 8 bis 30 C-Atomen können auch als alkoxylierte Fettalkohole bezeichnet werden. Beispiele für solche geeigneten alkoxylierten Fettalkohole sind die Verbindungen mit den INCI-Bezeichnungen Laureth-2 (Handelsprodukt Dehydol LS 2 H, Cognis), Laureth-3 (Handelsprodukt Dehydol LS 3 H, Cognis), Laureth-5, Laureth-8, Laureth-10 (Handelsprodukt Dehydol 100, Cognis), Laureth-12, Laureth-15, Laureth-20, Laureth-25, Laureth-30, Laureth-40, Laureth-50, Myreth-2, Myreth-3, Myreth-5, Myreth-8, Myreth-10, Myreth-12, Myreth-15, Myreth-20, Myreth-25, Myreth-30, Myreth-40, Myreth-50, Ceteth-2 (Handelsprodukt Brij C 2, Croda), Ceteth-3, Ceteth-5, Ceteth-7 (Handelsprodukt Nikkol BC 7, Nikko Chemicals), Ceteth-8, Ceteth-10 (Handelsprodukt Brij 56, Croda), Ceteth-12, Ceteth-15 (Handelsprodukt Nikkol BC 15 TX, Nikko Chemicals), Ceteth-20 (Handelsprodukt Brij C 20, Croda), Ceteth-25, Ceteth-30, Ceteth-40 (Handelsprodukt Nikkol BC 40 TX, Nikko Chemicals), Ceteth-50, Ceteth-150 (Handelsprodukt Nikkol BC 150, Nikko Chemicals), Steareth-2 (Handelsprodukt Nikkol S 2, Nikko Chemicals), Steareth-3, Steareth-5, Steareth-8, Steareth-10 (Handelsprodukt Eumulgin S 10, Cognis), Steareth-12, Steareth-15, Steareth-20 (Handelsprodukt Volpo S 20, Croda), Steareth-21 (Handelsprodukt Brij S 721, Croda), Steareth-25, Steareth-30, Steareth-40, Steareth-50, Ceteareth-2 (Handelsprodukt Lowenol C 279, Lowenstein), Ceteareth-3 (Handelsprodukt Hostacerin T 3, Clariant), Ceteareth-5, Ceteareth-8, Ceteareth-10, Ceteareth-12 (Handelsprodukt Eumulgin B 1, Cognis), Ceteareth-15, Ceteareth-20 (Handelsprodukt Eumulgin B 2, Cognis), Ceteareth-23 (Handelsprodukt Mergital C 23, Cognis), Ceteareth-25 (Handelsprodukt Eumulgin CS 25, Cognis), Ceteareth-30 (Handelsprodukt Eumulgin B 3, Cognis), Ceteareth-40, Ceteareth-50 (Handelsprodukt Mergital CS 50, Cognis), Oleth-2 (Handelsprodukt Nikkol BO 2, Nikko Chemicals), Oleth-3 (Handelsprodukt Volpo N 3, Croda), Oleth-5 (Handelsprodukt Volpo 5, Croda), Oleth-7 (Handelsprodukt Nikkol BO 2, Nikko Chemicals),Oleth-8, Oleth-10 (Handelsprodukt Volpo 10, Croda), Oleth-12, Oleth-15 (Handelsprodukt Nikkol BO 7, Nikko Chemicals), Oleth-20 (Handelsprodukt Nikkol BO 20, Nikko Chemicals), Oleth-25, Oleth-30, Oleth-40, Oleth-50 (Handelsprodukt Nikkol BO 50 V, Nikko Chemicals), Oleth-106 (Handelsprodukt Nikkol BO 106, Nikko Chemicals) sowie PPG-5-Laureth-5 (Handelsprodukt Eumulgin ES, Cognis), PPG-4-Laureth-5 (Handelsprodukt Marlox MO 154, Sasol).

Besonders bevorzugte alkoxylierte Fettalkohole sind Laureth-2, Ceteareth-12, Ceteareth-20, Ceteareth-30 und Ceteareth-50.

Anlagerungsprodukte von 1 bis 50 Mol Ethylenoxid und/oder 0 bis 5 Mol Propylenoxid an Alkylphenole mit 8 bis 15 C-Atomen in der Alkylgruppe können auch als alkoxylierte Alkylphenole bezeichnet werden. Beispiele für solche geeigneten alkoxylierten Alkylphenole sind die Verbindungen mit den INCI-Bezeichnungen Nonoxynol-1 (Handelsprodukt Emthox 6950, Cognis), Nonoxynol-2 (Handelsprodukt Igepal CO 210, Rhodia), Nonoxynol-4 (Handelsprodukt Tergitol NP 4, Dow), Nonoxynol-5 (Handelsprodukt Igepal CO 520, Rhodia), Nonoxynol-6 (Handelsprodukt Igepal CO 530, Rhodia), Nonoxynol-8 (Handelsprodukt Igepal CO 610, Rhodia), Nonoxynol-9 (Handelsprodukt Igepal CO 630, Rhodia), Nonoxynol-10 (Handelsprodukt Disponil 286, Cognis), Nonoxynol-12 (Handelsprodukt Igepal CO 720, Rhodia), Nonoxynol-15 (Handelsprodukt Igepal CO 730, Rhodia), Octoxynol-3 (Handelsprodukt Triton X 35, Dow), Octoxynol-5 (Handelsprodukt Triton X 45, Dow), Octoxynol-8 (Handelsprodukt Triton X 114, Dow), Octoxynol-9 (Handelsprodukt Triton X 100, Dow), Octoxynol-10 (Handelsprodukt Synperonic OP 10, Uniqema) und Octoxynol-13 (Handelsprodukt Triton X 102, Dow).

(Poly)glycerinfettsäureester und alkoxylierte (Poly)glycerinfettsäureester sind insbesondere Mono-und/oder Difettsäureglyceride, die gegebenenfalls Anlagerungsprodukte mit einem oder mehreren Äquivalenten Ethylenoxid sowie gegebenenfalls Propylenoxid bilden. Beispiele für geeignete (Poly)glycerinfettsäureester und alkoxylierte (Poly)glycerinfettsäureester sind gegebenenfalls alkoxylierte Fettsäurepartialglyceride, insbesondere die Verbindungen mit den INCI-Bezeichnungen Glyceryl Cocoate, Glyceryl Oleate (Handelsprodukt: Arlacel 186, Croda bzw. Monomuls 90 O 18, Cognis), Glyceryl Laurate (Handelsprodukt: Monomuls 90 L 12 Powder, Cognis), Glyceryl Myristate, Glyceryl Palmitate, Glyceryl Stearate (Handelsprodukt: Cutina GMS, Cognis; Tego Care 215, Goldschmidt: Mischung aus Glyceryl Stearate und Ceteareth-15), Glyceryl Distearate (Handelsprodukt: Lamesoft TM Benz, Cognis; Mischung aus Glyceryl Distearate, Coco Glucoside, Glyceryl Oleate, Glyceryl Stearate), sowie PEG-30 Glyceryl Laurate (Handelsprodukt: Tagat L, Goldschmidt), PEG-20 Glyceryl Laurate (Handelsprodukt: Tagat L 2, Goldschmidt), PEG-20 Glyceryl Stearate (Handelsprodukt: Tagat S 2, Goldschmidt), PEG-20 Glyceryl Oleate (Handelsprodukt: Tagat O 2, Goldschmidt) oder PEG-18 Glyceryl Oleate/Cocoate (Handelsprodukt: Antil 171, Goldschmidt).

Besonders bevorzugte (Poly)glycerinfettsäureester sind Glyceryl Stearate und Glyceryl Oleate.

Erfindungsgemäße Alkyl(poly)glucoside entsprechen der allgemeinen Formel RO-(Z)ₓ, wobei R für einen gesättigten oder ungesättigten Alkyl-Rest, bevorzugt für einen C₈ bis C₂₀-Alkyl-Rest, Z für einen Zuckerrest, wie Glucose, sowie x für die Anzahl der Zuckereinheiten steht. Bevorzugt steht x für eine Zahl von1 bis 5. Beispiele für geeignete Alkyl(poly)glucoside sind die Verbindungen mit den INCI-Bezeichnungen Decyl Glucoside (Handelsprodukt: Plantaren 2000 N, Cognis), Lauryl Glucoside (Handelsprodukt: Plantacare 1200, Cognis), Coco Glucoside (Handelsprodukt: Plantacare 818, Cognis; Lamesoft PO 65, Cognis: Mischung aus Coco Glucoside und Glyceryl Oleate), Capryl/Caprylyl Glucoside (Handelsprodukt: Oramix CG 110, Seppic), Stearyl Glucoside oder Cetearyl Glucoside (Handelsprodukt: Tego Care CG 90, Goldschmidt).

Besonders bevorzugte Alkyl(poly)glucoside sind die Verbindungen mit den INCI-Bezeichnungen Coco Glucoside, Lauryl Glucoside und Decyl Glucoside.

Eine Ausführungsform des ersten Erfindungsgegenstands ist ein Mittel, welches dadurch gekennzeichnet ist, dass das Mittel mindestens ein nichtionisches Tensid enthält, welches ausgewählt ist aus alkoxylierten Fettalkoholen und/oder (Poly)glycerinfettsäureestern und/oder alkoxylierten (Poly)-glycerinfettsäureestern und/oder Alkyl(poly)glucosiden.

Bevorzugt sind dabei alkoxylierte Fettalkoholen und Alkyl(poly)glucoside.

Eine Ausführungsform des ersten Erfindungsgegenstands ist daher ein Mittel, welches dadurch gekennzeichnet ist, dass das Mittel mindestens ein nichtionisches Tensid enthält, welches ausgewählt ist aus alkoxylierten Fettalkoholen und/oder Alkyl(poly)glucosiden.

Eine weitere Ausführungsform des ersten Erfindungsgegenstands ist ein Mittel, welches dadurch gekennzeichnet ist, dass das Mittel das und/oder die nichtionischen Tenside in einem Gewichtsanteil von 0,5 bis 25 Gew.-%, insbesondere von 1,0 bis 15 Gew.-%, und insbesondere 1,5 bis 10 Gew.-%, bezogen das Gesamtgewicht des anwendungsbereiten Mittels, enthält.

Als weiteren wesentlichen Bestandteil enthält das erfindungsgemäße Mittel als Oxidationsmittel mindestens Wasserstoffperoxid. Bevorzugt wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie Natriumpercarbamid, Polyvinylpyrrolidinon·n H₂O₂ (n ist eine positive ganze Zahl größer 0), Harnstoffperoxid und Melaminperoxid, eingesetzt werden.

Wasserstoffperoxid ist im anwendungsbereiten Mittel bevorzugt zu 0,1 bis 25 Gew.-%, insbesondere bevorzugt zu 1 bis 20 Gew.-% und besonders bevorzugt zu 4,5 bis 9 Gew.-%, jeweils berechnet auf 100%iges Wasserstoffperoxid und bezogen auf das Gesamtgewicht des anwendungsbereiten Mittels, enthalten.

Unter Berücksichtigung der bisher genannten bevorzugten Ausführungsformen stellt es eine ganz spezielle und ausdrücklich bevorzugte Ausführungsform dar, wenn das Mittel zum Aufhellen von keratinischen Fasern in einem kosmetischen Träger als erste Komponente mindestens eine Verbindung, ausgewählt aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methyl-pyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat, enthält,
als zweite Komponente mindestens ein anionisches Carbonsäure-haltiges Tensid, ausgewählt aus Verbindungen mit der INCI-Bezeichnung Sodium Lauryl Glucose Carboxylate, enthält,
als dritte Komponente mindestens ein nichtionisches Tensid, ausgewählt aus Verbindungen mit der INCI-Bezeichnung Coco Glucoside, Lauryl Glucoside, Glyceryl Stearate, Glyceryl Oleate, Laureth-2, Ceteareth-12 und Ceteareth-20, enthält
und als vierte Komponente Wasserstoffperoxid enthält.

### Besonders bevorzugt sind schließlich

Mittel, die 0,2 bis 4,0 Gew.-% 4-Acetyl-1-methylpyridinium-p-toluolsulfonat, 0,5 bis 10 Gew.-% Sodium Lauryl Glucose Carboxylate, 0,5 bis 15 Gew.-% Lauryl Glucoside sowie 2,0 bis 12,0 Gew.-% Wasserstoffperoxid enthalten,

Mittel, die 0,2 bis 4,0 Gew.-% 4-Acetyl-1-methylpyridinium-p-toluolsulfonat, 0,5 bis 10 Gew.-% Sodium Lauryl Glucose Carboxylate, 0,5 bis 15 Gew.-% Coco Glucoside sowie 2,0 bis 12,0 Gew.-% Wasserstoffperoxid enthalten,

Mittel, die 0,2 bis 4,0 Gew.-% 4-Acetyl-1-methylpyridinium-p-toluolsulfonat, 0,5 bis 10 Gew.-% Sodium Lauryl Glucose Carboxylate, 0,5 bis 15 Gew.-% Glyceryl Stearate sowie 2,0 bis 12,0 Gew.-% Wasserstoffperoxid enthalten,

Mittel, die 0,2 bis 4,0 Gew.-% 4-Acetyl-1-methylpyridinium-p-toluolsulfonat, 0,5 bis 10 Gew.-% Sodium Lauryl Glucose Carboxylate, 0,5 bis 15 Gew.-% Glyceryl Oleate sowie 2,0 bis 12,0 Gew.-% Wasserstoffperoxid enthalten,

Mittel, die 0,2 bis 4,0 Gew.-% 4-Acetyl-1-methylpyridinium-p-toluolsulfonat, 0,5 bis 10 Gew.-% Sodium Lauryl Glucose Carboxylate, 0,5 bis 15 Gew.-% Laureth-2 sowie 2,0 bis 12,0 Gew.-% Wasserstoffperoxid enthalten,

Mittel, die 0,2 bis 4,0 Gew.-% 4-Acetyl-1-methylpyridinium-p-toluolsulfonat, 0,5 bis 10 Gew.-% Sodium Lauryl Glucose Carboxylate, 0,5 bis 15 Gew.-% Ceteareth-12 sowie 2,0 bis 12,0 Gew.-% Wasserstoffperoxid enthalten,

Mittel, die 0,2 bis 4,0 Gew.-% 4-Acetyl-1-methylpyridinium-p-toluolsulfonat, 0,5 bis 10 Gew.-% Sodium Lauryl Glucose Carboxylate, 0,5 bis 15 Gew.-% Ceteareth-20 sowie 2,0 bis 12,0 Gew.-% Wasserstoffperoxid enthalten,
worin sich die Gewichtsanteile jeweils auf das Gesamtgewicht des anwendungsbereiten Mittels beziehen.

Zur optimalen Verbesserung des Griffgefühls des Haares hat es sich als besonders vorteilhaft erwiesen, wenn die den Griff verbessernden ausgewählten Tenside in einer ausreichenden Menge in dem erfindungsgemäßen Mittel vorhanden sind.

Zu einem besonders angenehmen Griffgefühl des Haares hat hierbei ein Gesamtgehalt von anionischen Carbonsäure-haltigen Tensid und nichtionischem Tensid in dem erfindungsgemäßen Mittel zum Aufhellen der Haare von in Summe mindestens 5 Gew.-%, bevorzugt mindestens 8 Gew.-%, und insbesondere mindestens 10 Gew.-%, bezogen auf das Gesamtgewicht des anwendungsbereiten Mittels, geführt.

Erfindungsgemäß kann das Mittel zusammen mit einem Katalysator auf das Haar aufgebracht werden, der die Oxidation der Farbstoffvorprodukte zusätzlich aktiviert. Solche Katalysatoren sind z. B. bestimmte Enzyme, lodide, Chinone oder Metallionen. Hierfür geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls bevorzugt sein. Besonders geeignet sind dabei Zn²⁺, Cu²⁺ und Mn²⁺.

Weiterhin hat es sich als vorteilhaft erweisen, wenn die Mittel mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-atomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Bei den pH-Werten im Sinne der vorliegenden Erfindung handelt es sich um pH-Werte, die bei einer Temperatur von 22 °C gemessen wurden.

Andererseits kann es aus Stabilitätsgründen sinnvoll sein, bestimmte Inhaltstoffe bei einem neutralen oder schwach sauren pH-Wert zu lagern und erst zum Zeitpunkt der Anwendung einem alkalischen Milieu auszusetzen. Zu diesen Inhaltstoffen gehört Wasserstoffperoxid, aber auch die kationischen Acylpyridiniumderivate der Formel (I) werden bevorzugt bei neutralen oder schwach sauren pH-Wert gelagert.

Es kann daher bevorzugt sein, wenn das erfindungsgemäße Mittel einen schwach sauren pH-Wert besitzt. Eine weitere Ausführungsform der vorliegenden Erfindung ist daher dadurch gekennzeichnet, dass das Mittel einen pH-Wert von pH 2 bis pH 6, bevorzugt vom pH 2,5 bis pH 4,5 besitzt.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus Ammoniak, anorganischen Salzen, insbesondere der Alkali- und Erdalkalimetalle, organischen Alkalisierungsmitteln, insbesondere Alkanolaminen, Aminen und basische Aminosäuren. Erfindungsgemäß bevorzugte Acidifizierungsmittel sind Genuss-Säuren, wie Milchsäure, Zitronensäure, Essigsäure, Äpfelsäure oder Weinsäure, verdünnte Mineralsäuren und deren in Wasser sauer reagierenden Salze sowie organische Phosphon- oder Sulfonsäuren.

Um die Aufhellleistung zu verbessern, besitzen die anwendungsbereiten Blondiermittel jedoch bevorzugt einen alkalischen pH-Wert. Daher ist es bevorzugt, ein Mittel des ersten Erfindungsgegenstands unmittelbar vor der Anwendung auf den keratinischen Fasern auf einen alkalischen pH-Wert zwischen 6 und 12, insbesondere zwischen 9 und 11, einzustellen.

Die erfindungsgemäß verwendbaren Alkalisierungsmittel werden bevorzugt aus der Gruppe, die gebildet wird aus Ammoniak, anorganischen Alkalisierungsmitteln, insbesondere der Alkali- und Erdalkalimetalle, organischen Alkalisierungsmitteln, insbesondere Alkanolaminen, Aminen und basische Aminosäuren.

Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Die basischen Aminosäuren werden bevorzugt ausgewählt aus der Gruppe, die gebildet wird aus Arginin, Lysin, Ornithin und Histidin.

Besonders bevorzugt als Alkalisierungsmittel enthält das Mittel jedoch Ammoniak. Eine Ausführungsform der vorliegenden Erfindung ist daher dadurch gekennzeichnet, dass das Mittel als Alkalisierungsmittel Ammoniak enthält.

Um Instabilitäten aufgrund von unerwünschten Reaktionen zwischen den einzelnen Inhaltsstoffen während der Lagerung der Mittel zu vermeiden, ist es zweckmäßig, die Inhaltsstoffe erst unmittelbar vor der Anwendung miteinander in Kontakt zu bringen. Dazu ist es zweckmäßig, die Inhaltsstoffe getrennt voneinander zu konfektionieren.

Es ist insbesondere von Vorteil, Alkalisierungsmittel und Wasserstoffperoxid getrennt zu lagern. Das Acylpyridiniumderivat der Formel (I) wie auch das anionische, Carbonsäure-haltige Tensid und das nichtionische Tensid können sowohl zusammen mit Wasserstoffperoxid wie auch zusammen mit dem Alkalisierungsmittel konfektioniert werden.

Das anwendungsbereite, erfindungsgemäße Mittel wird vor der Anwendung durch Vermischen einer Alkalisierungszubereitung M1 und einer Oxidationszubereitung M2 hergestellt. Es ist daher vorteilhaft, dem Anwender beide Zubereitungen in einem Set anzubieten. Daher ist eine bevorzugte Darreichungsform des anwendungsbereiten Mittels eine getrennte Verpackungseinheit, worin die Mittel M1 und M2 jeweils getrennt voneinander verpackt vorliegen.

Ein weiterer Gegenstand der vorliegenden Erfindung ist daher eine Mehrkomponentenverpackungseinheit (Kit-of-Parts), umfassend mindestens zwei getrennt voneinander konfektionierte Container, wobei
ein erster Container C1 mindestens ein Mittel M1, enthaltend in einem kosmetisch verträglichen Träger als Oxidationsmittel mindestens Wasserstoffperoxid, und
ein zweiter Container C2 mindestens ein Mittel M2, enthaltend in einem kosmetisch verträglichen Träger mindestens ein Alkalisierungsmittel, mindestens ein nichtionisches Tensid und mindestens ein anionisches, Carbonsäure-haltiges Tensid, beinhaltet,
dadurch gekennzeichnet, dass mindestens eines der Mittel M1 und/oder M2 ein Acylpyridiniumderivat der Formel (I) gemäß dem ersten Erfindungsgegenstand enthält.

Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Weiterhin kann es erfindungsgemäß besonders vorteilhaft sein, wenn das genannte Kit-of-Parts mindestens ein weiteres Haarbehandlungsmittel in einem getrennten Container enthält, insbesondere eine Konditioniermittelzubereitung oder ein Blondierpulver mit Peroxodisulfaten. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Erfindungsgemäße, anwendungsbereite Mittel sind vorzugsweise wässrige, fließfähige Zubereitungen. Die erfindungsgemäßen Mittel können weiterhin alle für solche Zubereitungen bekannten Wirk-, Zusatz- und Hilfsstoffe enthalten. Die anwendungsbereiten Mittel als Mischung aus Mittel M1 und M2 können dabei weitere oberflächenaktive Substanzen, ausgewählt aus zusätzlichen anionischen, zwitterionischen, amphoteren und kationischen Tensiden enthalten.

Zusätzliche anionische Tenside sind gekennzeichnet durch eine wasserlöslich machende, anionische Gruppe wie eine Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Beispiele solcher zwitterionischen Tenside sind die Betaine wie die N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline mit jeweils 8 bis 18 C-Atomen in der Alkyl- oder Acylgruppe sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Übliche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat und C₁₂-C₁₈-Acylsarcosin.

Erfindungsgemäß bevorzugt sind in anwendungsbereiten Mitteln kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyl-trimethylammoniumchloride, Dialkyldimethylammoniumchloride und Trialkylmethylammoniumchloride sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Alkylamidoamine werden üblicherweise durch Amidierung natürlicher oder synthetischer Fettsäuren und Fettsäureschnitte mit Dialkylaminoaminen hergestellt, wie Stearamidopropyl-dimethylamin. Ebenfalls bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-Bis(2-Palmitoyloxyethyl)dimethylammoniumchlorid, sowie Dehyquart F-75, Dehyquart C-4046, Dehyquart L80 und Dehyquart AU-35 sind Beispiele für solche Esterquats. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können zwitterionische und/oder amphotere Tenside sowie deren Mischungen bevorzugt sein.

Die erfindungsgemäßen Mittel ebenso wie die Mittel M1 und/oder M2 können weitere Wirk-, Hilfs- und Zusatzstoffe enthalten. Weitere, erfindungsgemäß einsetzbare Wirk-, Hilfs- und Zusatzstoffe sind beispielsweise anionische Polymere (wie Carbomere, Co- und Crosspolymere von Acrylsäure, Methacrylsäure, Maleinsäure, Itaconsäure und gegebenenfalls weiteren nichtionischen Monomeren); kationische Polymere (wie Polyquaternium-4, Polyquaternium-10, Polyquaternium-24, Polyquaternium-67, Polyquaternium-72, Polyquaternium-75, Polyquaternium-29, Polyquaternium-6, Polyquaternium-7, Polyquaternium-22); nichtionische Polymere (wie Vinylpyrrolidinon/Vinylacrylat-Copolymere, Polyvinylpyrrolidinon und Vinylpyrrolidinon/Vinylacetat-Copolymere und Polysiloxane); zwitterionische und amphotere Polymere (wie Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere); Siliconen (wie Dimethiconen, Dimethiconcopolyolen, Polyalkylsiloxanen, Polyalkylarylsiloxanen, Dimethiconolen, Cyclomethiconen und Amodimethiconen); Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol); Strukturanden (wie Zucker, Maleinsäure und Milchsäure) und Konsistenzgeber (wie Zuckerester, Polyolester oder Polyolalkylether); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren); Parfümöle; Pflegeöle; Cyclodextrine; Entschäumer wie Silicone; Farbstoffe und Pigmente zum Anfärben des Mittels; Antischuppenwirkstoffe (wie Piroctone Olamine, Zink Omadine und Climbazol); Lichtschutzmittel (insbesondere derivatisierte Benzophenone, Zimtsäure-Derivate und Triazine); Wirkstoffe (wie Allantoin, Pyrrolidoncarbonsäuren, Cholesterin und deren Salze); weitere Fette und Wachse (wie Fettalkohole, Bienenwachs, Montanwachs und Paraffine); Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/- Acrylamid-Copolymere); Perlglanzmittel (wie Ethylenglykolmono- und -distearat sowie PEG-3-distearat); Treibmittel (wie Propan-Butan-Gemische, N₂O, Dimethylether, CO₂ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel nicht nur als reine Aufhellmittel, d.h. als sogenannte Blondiermittel, sondern auch als mattierende Aufhellmittel bereitgestellt werden, die gleichzeitig mit der Aufhellung auch eine Mattierung der Keratinfasern bewirken, so dass die bei Blondierungen häufig auftretenden, aber unerwünschten Farbverschiebungen in rötliche oder orange Bereiche durch eine geringe Färbung, insbesondere in kühlen Farbtönen, ausgeglichen wird.

Für solche Mattierungen werden als färbende Komponente sogenannte direktziehende Farbstoffe eingesetzt. Hierbei handelt es sich um Farbstoffmoleküle, die direkt auf das Substrat aufziehen und keinen oxidativen Prozess zur Ausbildung der Farbe benötigen.

In einer Ausführungsform der vorliegenden Erfindung können die erfindungsgemäßen, anwendungsbereiten Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 5,0 Gew.-%, bevorzugt von 0,001 bis 1,5 Gew.-%, jeweils bezogen auf die gesamte Anwendungszubereitung, eingesetzt. Die Gesamtmenge an direktziehenden Farbstoffen beträgt vorzugsweise höchstens 1,0 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Kationische direktziehende Farbstoffe, die unter dem Warenzeichen Arianor^{®} vertrieben werden, sind erfindungsgemäß ebenfalls besonders bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87 oder Tetrabromphenolblau und Acid Red 51.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in handelsüblichen Blondiermittel in der Regel eine Kombination aus Wasserstoffperoxid und Persulfaten bzw. Peroxodisulfaten eingesetzt. Es hat sich gezeigt, dass die Zugabe der erfindungsgemäßen Acylpyridiniumderivate der Formel (I) in Kombination mit einem anionischen Carbonsäure-haltigen Tensid und einem nichtionischen Tensid bereits zu einer deutlichen Verbesserung der Aufhellleistung führt, so dass in der Regel auf die Zugabe von Persulfaten verzichtet werden kann.

Es kann jedoch, sollte der Verbraucher den Wunsch nach einer sehr starken Blondierung verspüren, in einer weiteren Ausführungsform bevorzugt sein, wenn neben Wasserstoffperoxid, der kationischen Acylpyridiniumverbindung der Formel (I), einem anionischen Carbonsäure-haltigen Tensid und einem nichtionischen Tensid zusätzlich mindestens ein anorganisches Persulfatsalz bzw. Peroxodisulfatsalz in dem Mittel zum Aufhellen der keratinischen Fasern enthalten ist.

Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Diese Peroxodisulfate werden dem Mittel ebenfalls bevorzugt erst unmittelbar vor der Anwendung beigemischt, um Instabilitäten bei der Lagerung zu vermeiden.

Ein weiterer Erfindungsgegenstand ist ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand die beiden Mittel M1 und M2 innig miteinander vermischt werden, und das resultierende, anwendungsbereite Farbveränderungsmittel anschließend auf die Fasern aufgebracht wird, für eine Einwirkdauer von 5 bis 60 min, bevorzugt 30 bis 45 min auf den Fasern belassen und schließlich ausgespült wird.

Die Anwendungstemperaturen können in einem Bereich zwischen 15 und 40 °C liegen. Nach der Einwirkungszeit wird das verbleibende Mittel durch Ausspülen von dem Haar entfernt. Das Nachwaschen mit einem Shampoo entfällt, wenn ein stark tensidhaltiger Träger verwendet wurde.

Im Rahmen dieses Gegenstandes der Erfindung gelten mutatis mutandis die oben getroffenen Aussagen analog.

Ein weiterer Gegenstand der vorliegenden Erfindung ist die kosmetische Verwendung eines Mittels des ersten Erfindungsgegenstands zum Aufhellen von keratinhaltigen Fasern, insbesondere menschlichen Haaren.

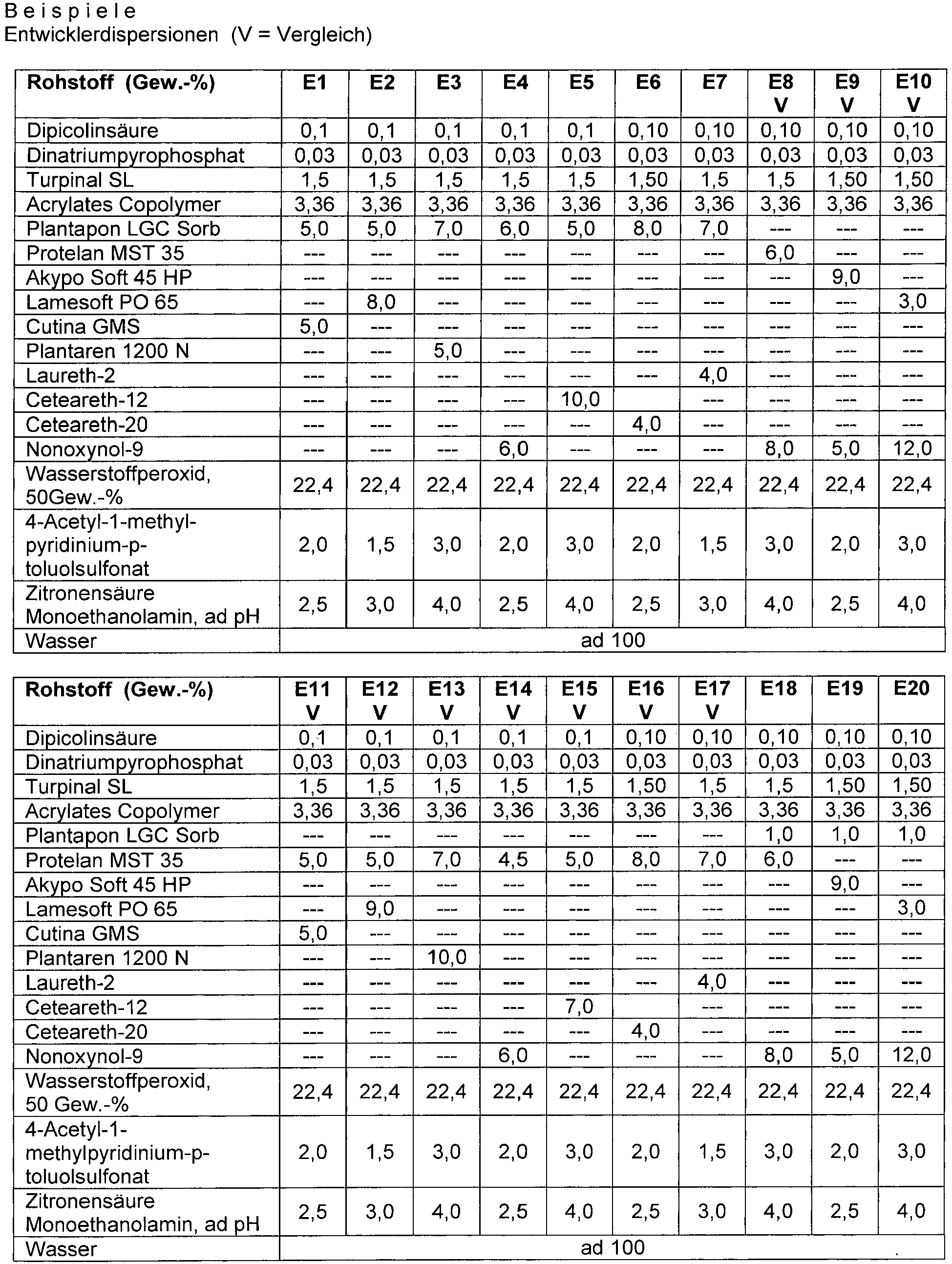

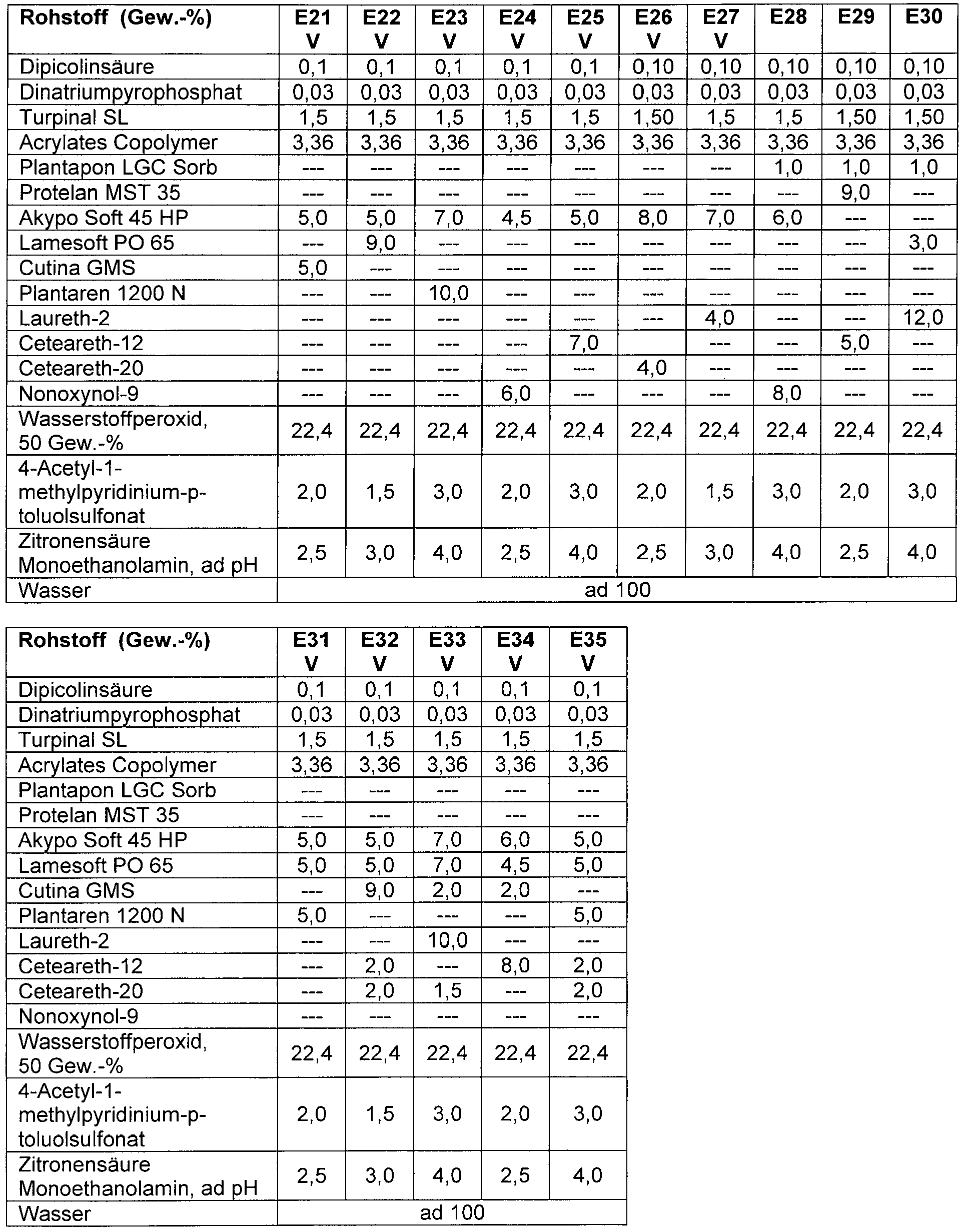

| | |
|---|---|
| Turpinal SL | ca. 60% Aktivstoff; INCI-Bezeichnung: Etidronic Acid (Thermophos) |
| Plantapon LGC Sorb | ca. 28,5-34% Aktivstoff; INCI-Bezeichnung: Lauryl Glucose Carboxylate (ca. 15-25%), Lauryl Glucoside (ca. 10-20%) (Cognis) |
| Protelan MST 35 | ca. 35% Aktivstoff; INCI-Bezeichnung: Sodium Myristoyl Sarcosinate, Sodium Cocoyl Methyl Taurate (Zschimmer&Schwarz) |
| Akypo Soft 45 HP | ca. 21% Aktivstoff; INCI-Bezeichnung: Sodium Laureth-6 Carboxylate (Kao) |
| Lamesoft PO 65 | ca. 66% Aktivstoff; INCI-Bezeichnung: Coco Glucoside, Glyceryl Oleate (Cognis) |
| Cutina GMS | INCI-Bezeichnung: Glyceryl Stearate (Cognis) |
| Plantaren 1200 N | ca. 50-60% Aktivstoff; INCI-Bezeichnung: Lauryl Glucoside (Cognis) |

Die auf dieser Weise erhaltenen Entwicklerdispersionen E1 bis E35 wurden im Verhältnis 1:1 mit der folgenden Blondiercreme B0 vermischt.

| **Rohstoff (Gew.-%)** | **B0** |
|---|---|
| Cetearyl Alkohol | 12,00 |
| Cocosfettalkohol | 2,40 |
| Texapon NSO | 26,50 |
| Stabylen 30 | 0,10 |
| Decyloleat | 2,40 |
| Turpinal SL | 0,20 |
| Natriumsilikat 40/42 | 0,50 |
| Ascorbinsäure | 0,20 |
| Wasser | ad 100 |

### Eingesetzte Handelsprodukte:

- Stabylen 30: INCI-Bezeichnung: Acrylates/ Vinyl Isodecanoate Crosspolymer (Sigma)

### 2. Bereitstellung des erfindungsgemäßen Mittels in zwei Komponenten Entwicklerdispersionen, enthaltend Oxidationsmittel und Bleichaktivator

Es wurde die folgende Entwicklerdispersion hergestellt

| **Rohstoff (Gew.-%)** | **E 0a** | **E 0b** |
|---|---|---|
| Ammoniak 25 % | 0,62 | 0,62 |
| Dipicolinsäure | 0,10 | 0,10 |
| Dinatriumpyrophosphat | 0,03 | 0,03 |
| Turpinal SL | 1,50 | 1,50 |
| Texapon NSO | 2,00 | 2,00 |
| Acrylates Copolymer | 3,36 | 3,36 |
| Wasserstoffperoxid, 50 Gew.-% | 22,40 | 22,40 |
| 4-Acetyl-1-methylpyridinium-p-toluol-sulfonat | --- | 2,00 |
| Wasser | ad 100 | ad 100 |

Diese Entwicklerdispersionen wurden jeweils im Verhältnis 1:1 mit den folgenden Blondiercremes B1 bis B35 vermischt:

Blondiercremes, enthaltend ein anionisches Carbonsäure-haltiges Tensid und ein nichtionisches Tensid:

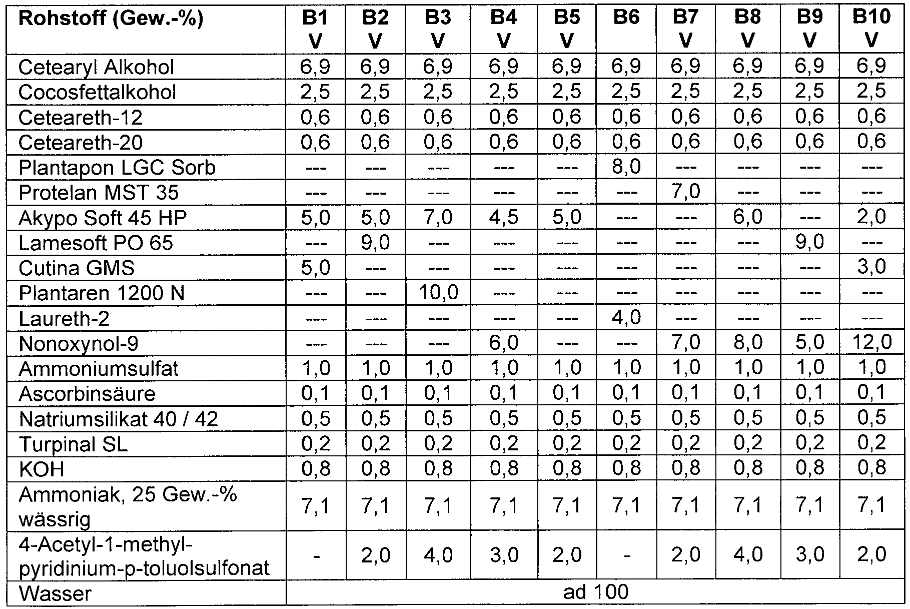

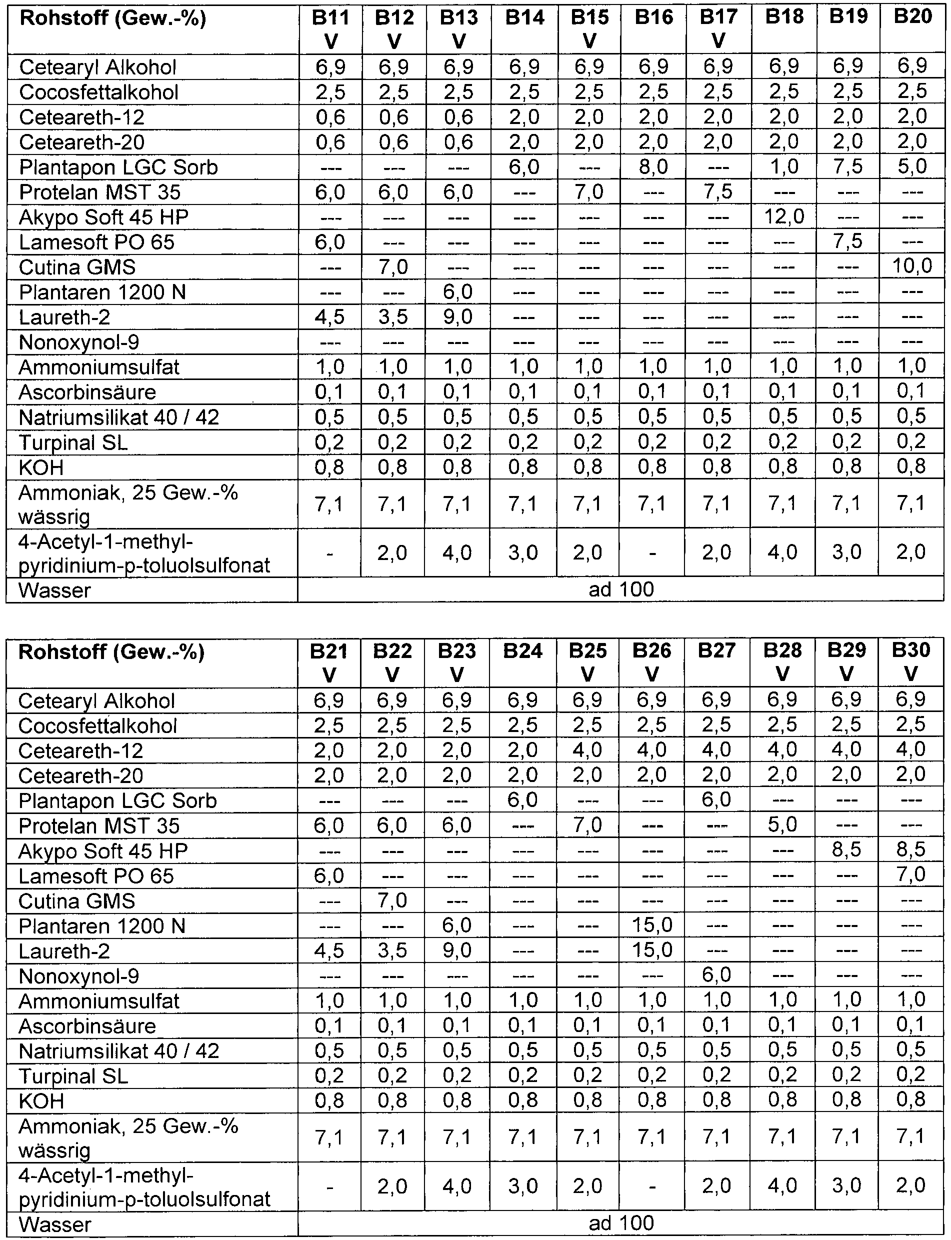

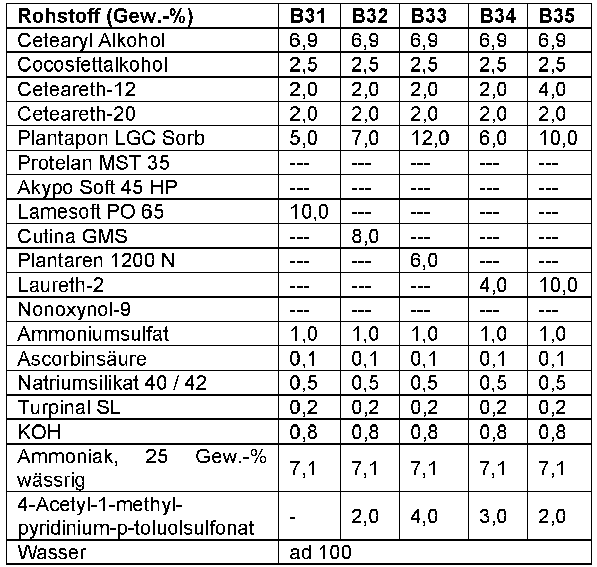

## Patentansprüche

1. Mittel zum Aufhellen von keratinischen Fasern, enthaltend in einem kosmetisch verträglichen Träger, mindestens ein anionisches, Carbonsäure-haltiges Tensid, mindestens ein nichtionisches Tensid und mindestens Wasserstoffperoxid, wobei das Mittel zusätzlich mindestens ein Acylpyridiniumderivat der Formel (I), 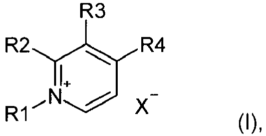 worin
R1 für eine C₁-C₆-Alkylgruppe, eine C₂-C₆-Alkenylgruppe, eine C₂-C₆-Hydroxyalkyl-gruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
R2, R3 und R4 jeweils unabhängig voneinander für Wasserstoff, eine C₁-C₆-Alkylgruppe, ein Halogenatom oder eine C₁-C₆-Acylgruppe steht, mit der Massgabe, dass zumindest einer der Reste R2, R3 und R4 für eine C₁-C₆-Acylgruppe steht,
X⁻ für ein physiologisch verträgliches Anion steht, enthält,
**dadurch gekennzeichnet, dass**
das Mittel mindestens ein anionisches, Carbonsäure-haltiges Tensid enthält, welches ausgewählt ist aus den Alkylglucosidcarbonsäuren.

2. Mittel nach Anspruch 1, **dadurch gekennzeichnet, dass** es mindestens ein Acylpyridiniumderivat der Formel (I) enthält, das ausgewählt wird aus mindestens einer Verbindung der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat, insbesondere 4-Acetyl-1-methylpyridinium-p-toluolsulfonat.

3. Mittel nach einem der Ansprüche 1 bis 2, **dadurch gekennzeichnet, dass** die Acylpyridiniumderivate der Formel (I) in einem Gewichtsanteil von 0,1 bis 10 Gew.-%, insbesondere von 0,2 bis 4 Gew.-%, und insbesondere 0,5 bis 2 Gew.-%, jeweils bezogen das Gesamtgewicht des Mittels, enthalten sind.

4. Mittel nach einem der Ansprüche 1 bis 3, **dadurch gekennzeichnet, dass** das Mittel als anionisches, Carbonsäure-haltiges Tensid die Alkylglucosidcarbonsäure Lauryl Glucose Carboxylate enthält.

5. Mittel nach einem der Ansprüche 1 bis 4, **dadurch gekennzeichnet, dass** das Mittel mindestens das und/oder die anionischen, Carbonsäure-haltigen Tenside in einem Gewichtsanteil von 0,5 bis 25 Gew.-%, insbesondere von 1,0 bis 15 Gew.-%, und insbesondere 1,5 bis 10 Gew.-%, bezogen das Gesamtgewicht des anwendungsbereiten Mittels, enthält.

6. Mittel nach einem der Ansprüche 1 bis 5, **dadurch gekennzeichnet, dass** das Mittel mindestens ein nichtionisches Tensid enthält, welches ausgewählt ist aus alkoxylierten Fettalkoholen und/oder (Poly)glycerinfettsäureestern und/oder alkoxylierten (Poly)glycerinfettsäureestern und/oder Alkyl-(poly)glucosiden, bevorzugt ausgewählt ist aus alkoxylierten Fettalkoholen und/oder Alkyl(poly)-glucosiden.

7. Mittel nach einem der Ansprüche 1 bis 6, **dadurch gekennzeichnet, dass** das Mittel mindestens das und/oder die nichtionischen Tenside in einem Gewichtsanteil von 0,5 bis 25 Gew.-%, insbesondere von 1,0 bis 15 Gew.-%, und insbesondere 1,5 bis 10 Gew.-%, bezogen das Gesamtgewicht des anwendungsbereiten Mittels, enthält.

8. Kosmetische Verwendung eines Mittels nach einem der Ansprüche 1 bis 7 zum Aufhellen von keratinhaltigen Fasern, insbesondere menschlichen Haaren.

## Claims

1. An agent for lightening keratinic fibers, containing, in a cosmetically acceptable carrier, at least one anionic, carboxylic-acid-containing surfactant, at least one non-ionic surfactant and at least hydrogen peroxide, the agent additionally containing at least one acylpyridinium derivative of formula (I), 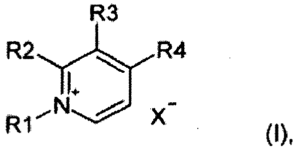 where
R1 is a C₁-C₆ alkyl group, a C₂-C₆ alkenyl group, a C₂-C₆ hydroxyalkyl group, a C₁-C₆ alkoxy C₂-C₆ alkyl group, a carboxy C₂-C₆ alkyl group, an aryl C₁-C₆ alkyl group, a heteroaryl C₁-C₆ alkyl group, an aryl group or a heteroaryl group,
R2, R3 and R4 are each, independently of one another, hydrogen, a C₁-C₆ alkyl group, a halogen atom or a C₁-C₆ acyl group, provided that at least one of the functional groups R2, R3 and R4 is a C₁-C₆ acyl group,
X- is a physiologically acceptable anion,
**characterized in that**
the agent contains at least one anionic, carboxylic-acid-containing surfactant which is selected from the alkyl glucoside carboxylic acids.

2. The agent according to claim 1, **characterized in that** it contains at least one acylpyridinium derivative of formula (I) which is selected from at least one compound of the group formed by 4-acetyl-1-methylpyridinium-p-toluenesulfonate, 4-acetyl-1-methylpyridinium-benzenesulfonate, 4-acetyl-1-methylpyridinium hydrogen sulfate, 4-acetyl-1-methylpyridinium acetate, 4-acetyl-1-allylpyridinium-p-toluenesulfonate, 4-acetyl-1-allylpyridinium-benzenesulfonate, 4-acetyl-1-allylpyridinium hydrogen sulfate, 4-acetyl-1-allylpyridinium acetate, 2-acetyl-1-methylpyridinium-p-toluenesulfonate, 2-acetyl-1-methylpyridinium-benzenesulfonate, 2-acetyl-1 methylpyridinium hydrogen sulfate, 2-acetyl-1-methylpyridinium acetate, 2-acetyl-1-allylpyridinium-p-toluenesulfonate, 2-acetyl-1-allylpyridinium-benzenesulfonate, 2-acetyl-1-allylpyridinium hydrogen sulfate and 2-acetyl-1-allylpyridinium acetate, in particular 4-acetyl-1-methylpyridinium-p-toluenesulfonate.

3. The agent according to claim 1 or 2, **characterized in that** the acylpyridinium derivatives of formula (I) are contained in a percentage by weight of from 0.1 to 10 wt.%, in particular 0.2 to 4 wt.% and in particular 0.5 to 2 wt.%, in each case based on the total weight of the agent.

4. The agent according to one of claims 1 to 3, **characterized in that** the agent contains the alkyl glucoside carboxylic acid lauryl glucose carboxylate as the anionic, carboxylic-acid-containing surfactant.

5. The agent according to one of claims 1 to 4, **characterized in that** the agent contains at least the anionic, carboxylic acid-containing surfactant and/or surfactants in a percentage by weight of from 0.5 to 25 wt.%, in particular 1.0 to 15 wt.% and in particular 1.5 to 10 wt.%, based on the total weight of the ready-to-apply agent.

6. The agent according to one of claims 1 to 5, **characterized in that** the agent contains at least one non-ionic surfactant which is selected from alkoxylated fatty alcohols and/or (poly)glycerol fatty acid esters and/or alkoxylated (poly) glycerol fatty acid esters, preferably selected from alkoxylated fatty alcohols and/or alkyl(poly)-glucosides.

7. The agent according to one of claims 1 to 6, **characterized in that** the agent contains at least the non-ionic surfactant and/or surfactants in a percentage by weight of from 0.5 to 25 wt.%, in particular 1.0 to 15 wt.% and in particular 1.5 to 10 wt.%, based on the total weight of the ready-to-apply agent.

8. The cosmetic use of an agent according to one of claims 1 to 7 for lightening keratin-containing fibers, in particular human hair.

## Revendications

1. Produit d'éclaircissement de fibres de kératine contenant, dans un support cosmétiquement compatible, au moins un tensioactif anionique contenant un acide carboxylique, au moins un tensioactif non-ionique et au moins un peroxyde d'hydrogène, le produit contenant en plus au moins un dérivé d'acylpyridinium de formule (I) : 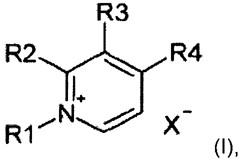 où
R1 représente un groupe C₁₋₆-alkyle, un groupe C₂₋₆-alcényle, un groupe C₂₋₆-hydroxyalkyle, un groupe C₂₋₆-alcoxy-C₂₋₆-alkyle, un groupe carboxy-C₂₋₆-alkyle, un groupe aryl-C₁₋₆-alkyle, un groupe hétéroaryl-C₁₋₆-alkyle, un groupe aryle ou un groupe hétéroaryle,
R2, R3 et R4 représentent respectivement, indépendamment les uns des autres, un hydrogène, un groupe C₁₋₆-alkyle, un halogène ou un groupe C₁₋₆-acyle, à condition qu'au moins un des résidus R2, R3 et R4 représente un groupe C₁₋₆-acyle,
X⁻ représente un anion physiologiquement compatible,
**caractérisé en ce que** le produit contient au moins un tensioactif anionique contenant de l'acide carboxylique choisi parmi les acides alkylglucosidecarboxyliques.

2. Produit selon la revendication 1, **caractérisé en ce qu'**il contient au moins un dérivé d'acylpyridinium de formule (I) choisi parmi au moins un composé du groupe constitué du 4-acétyl-1-méthylpyridinium-paratoluènesulfonate, 4-acétyl-1-méthylpyridinium-benzènesulfonate, 4-acétyl-1-méthylpyridinium-hydrogénosulfate, 4-acétyl-1-méthylpyridinium-acétate, 2-acétyl-1-méthylpyridinium-paratoluènesulfonate, 2-acétyl-1-méthylpyridinium-benzènesulfonate, 2-acétyl-1-méthylpyridinium-hydrogénosulfate, 2-acétyl-1-méthylpyridinium-acétate, 2-acétyl-1-allylpyridinium-paratoluènesulfonate, 2-acétyl-1-allylpyridinium-benzènesulfonate, 2-acétyl-1-allylpyridinium-hydrogénosulfate, 2-acétyl-1-allylpyridinium-acétate, en particulier du 4-acétyl-1-méthylpyridinium-paratoluènesulfonate.

3. Produit selon la revendication 1 ou 2, **caractérisé en ce que** les dérivés d'acylpyridinium de formule (I) sont contenus à hauteur d'une teneur pondérale de 0,1 à 10 % en poids, en particulier de 0,2 à 4 % en poids et en particulier de 0,5 à 2 % en poids, respectivement rapporté au poids total du produit.

4. Produit selon la revendication 1 à 3, **caractérisé en ce que** le produit contient comme tensioactif anionique contenant un acide carboxylique l'acide alkylglucosidecarboxylique lauryl glucose carboxylate.

5. Produit selon la revendication 1 à 4, **caractérisé en ce que** le produit contient l'au moins un tensioactif anionique contenant un acide carboxylique à hauteur de 0,5 à 25 % en poids, en particulier de 1,0 à 15 % en poids et en particulier de 1,5 à 10 % en poids, rapporté au poids total du produit prêt à l'emploi.

6. Produit selon la revendication 1 à 5, **caractérisé en ce que** le produit contient au moins un tensioactif non-ionique choisi parmi des alcools gras alcoxylés et/ou des esters d'acides gras (poly)glycérinés et/ou des esters d'acides gras (poly)glycérinés alcoxylés et/ou des alkyl-(poly)glucosides, choisi préférentiellement parmi des alcools gras alcoxylés et/ou des alkyl-(poly)glucosides.

7. Produit selon la revendication 1 à 6, **caractérisé en ce que** le produit contient l'au moins un tensioactif non-ionique à hauteur de 0,5 à 25 % en poids, en particulier de 1,0 à 15 % en poids et en particulier de 1,5 à 10 % en poids, rapporté au poids total du produit prêt à l'emploi.

8. Utilisation cosmétique d'un produit selon une des revendications 1 à 7 pour éclaircir des fibres de kératine, en particulier des cheveux humains.
